# FASCICULE DE BREVET EUROPEEN

(11) **EP 3 013 862 B1**
(45) Date de publication et mention de la délivrance du brevet: **18.07.2018**
(21) Numéro de dépôt: 14749901.6
(22) Date de dépôt: 27.06.2014
(51) Int. Cl.: C07K 16/28, G01N 33/50

(54) **MODULATEURS DU CANAL SODIQUE NAV1.9 ET SA DÉTECTION EN METHODES DIAGNOSTIQUES D'UNE MALADIE CUTANÉE INFLAMMATOIRE**
MODULATOREN DES NAV1.9 NATRIUMKANALS UND SEINE DETEKTION IM DIAGNOSEVERFAHREN VON ENTZÜNDLICHEN HAUTKRANKHEITEN
MODULATORS OF THE NAV1.9 SODIUM CHANNEL AND ITS DETECTION IN DIAGNOSTIC METHODS OF AN INFLAMMATORY SKIN DISEASE

(30) Priorité: 28.06.2013 FR 1356349
(43) Date de publication de la demande: 04.05.2016
(73) Titulaire: Galderma Research & Development, 06410 Biot (FR); Centre National de la Recherche Scientifique, 75016 Paris (FR); Université d'Aix-Marseille, 13007 Marseille (FR)
(72) Inventeur: CREST, Marcel, 13006 Marseille (FR); DELMAS, Patrick, 13880 Velaux (FR); LONIGRO-RAME, Aurélie, 13820 Ensues la Redonne (FR); OSORIO, Nancy, 83240 Cavalaire sur Mer (FR)
(74) Mandataire: Cabinet Becker et Associés
(86) Numéro de dépôt international: PCT/FR2014/051642
(87) Numéro de publication internationale: WO 2014/207401

(56) Documents cités:
- WO-A2-2007/023298
- STRICKLAND IAIN T ET AL: "Changes in the expression of NaV1.7, NaV1.8 and NaV1.9 in a distinct population of dorsal root ganglia innervating the rat knee joint in a model of chronic inflammatory joint pain.", EUROPEAN JOURNAL OF PAIN JUL 2008, vol. 12, no. 5, juillet 2008 (2008-07), pages 564-572, XP002720204, LONDON, ENGLAND ISSN: 1532-2149
- YU YAO-QING ET AL: "Antisense-mediated knockdown of Na(V)1.8, but not Na(V)1.9, generates inhibitory effects on complete Freund's adjuvant-induced inflammatory pain in rat.", PLOS ONE 2011, vol. 6, no. 5, E19865, 2011, pages 1-9, XP002731217, ISSN: 1932-6203
- PADILLA ET AL: "Expression and localization of the Nav1.9 sodium channel in enteric neurons and in trigeminal sensory endings: Implication for intestinal reflex function and orofacial pain", MOLECULAR AND CELLULAR NEUROSCIENCES, vol. 35, no. 1, 26 avril 2007 (2007-04-26) , pages 138-152, XP022056623, SAN DIEGO, US ISSN: 1044-7431, DOI: 10.1016/J.MCN.2007.02.008
- LOLIGNIER ET AL: "Nav1.9 channel contributes to mechanical and heat pain hypersensitivity induced by subacute and chronic inflammation", PLOS ONE, vol. 6, no. 8, E23083, 1 août 2011 (2011-08-01), pages 1-11, XP002698323,
- FRANK H YU ET AL: "Overview of the voltage-gated sodium channel family", GENOME BIOLOGY (ONLINE), BIOMED CENTRAL LTD, GB, vol. 4, no. 3, 1 janvier 2003 (2003-01-01) , page 207, XP002415109, ISSN: 1465-6914

## Description

### Domaine de l'invention

L'invention concerne le diagnostic des maladies dermatologiques inflammatoires, en particulier la rosacée.

### Contexte de l'invention

Les maladies cutanées inflammatoires englobent de nombreuses pathologies telles que la rosacée, le psoriasis, l'eczéma de contact, la dermatite atopique, ou encore le prurit.
La rosacée est une dermatose inflammatoire commune chronique et progressive liée à des désordres vasculaires. Elle affecte principalement la partie centrale du visage et se caractérise par un rougissement du visage accompagné de bouffées de chaleur, un érythème facial, de papules, de pustules, de télangiectasie et parfois de lésions oculaires appelées rosacée oculaire. Dans des cas extrêmes, particulièrement chez l'homme, on observe une hypertrophie au niveau nasal appelé rhinophyma. La rosacée survient entre 25 et 70 ans et évolue sur plusieurs années avec des phases de rémission et des phases d'exacerbation. La rosacée est beaucoup plus commune chez les personnes au teint clair et touche particulièrement les femmes. Cependant, les atteintes les plus sévères sont généralement observées chez les hommes. La rosacée est classifiée en 4 sous-types en fonction de diverses caractéristiques cliniques (Wilkin J et al, JAAD, 2002, 46: 584-587) : la rosacée érythématotélangiectasique (sous-type 1), la rosacée papulopustulaire (sous-type 2), la rosacée phymateuse (sous-type 3) et la rosacée oculaire (sous-type 4). Il existe également des formes plus rares de rosacée comme la variante granulomateuse qui est caractérisée par des papules ou nodules indurés jaunes, bruns ou rouges, et par des lésions monomorphes à l'endroit des papules.

Les signes pathologiques de la rosacée varient en fonction du sous-type de la maladie. Néanmoins, on note que les réactions inflammatoires locales et l'hyperactivité vasculaire sont des manifestations constantes de la rosacée.

La pathogénèse de la rosacée est mal connue et peut impliquer plusieurs facteurs. La maladie peut être causée ou favorisée par la présence de microorganismes folliculaires tels que des bactéries et les acariens *Demodex Folliculorum*, une réponse immunitaire innée aberrante, une réactivité vasculaire anormale et une hypersensibilité à des stimuli environnementaux tels que l'exposition aux UVs, les brusques changements de température, la consommation de boissons chaudes, de plats épicés et d'alcool, les fortes émotions (stress, gêne, colère...).

Classiquement, la rosacée est traitée oralement ou par voie topique par des antibiotiques tels que les tétracyclines, l'érythromycine, la clindamycine, mais aussi par la vitamine A, l'acide salicylique, l'acide azélaique, le sulfacétamide de soufre, le métronidazole ou par l'isotrétinoïne dans les formes sévères. Les thérapies disponibles peuvent présenter des effets secondaires désagréables pour le patient tels que des irritations et leur efficacité peut être limitée vis-à-vis de certains symptômes ou sous-type de rosacée. Enfin, ces traitements visent généralement les symptômes de la maladie sans en traiter la cause.

Le psoriasis est une dermatose chronique, évoluant par poussée ou par crise, qui touche environ 2% de la population. Le psoriasis est caractérisé par une hyperprolifération épidermique (renouvellement de l'épiderme accéléré) associée à des troubles de la kératinisation. Les lésions psoriasiques se présentent généralement sous la forme de plaques érythémato-squameuses, souvent prurigineuses. On observe généralement au niveau des lésions une infiltration leucocytaire et une inflammation modérée du derme et de l'épiderme, ce qui laisse penser que le psoriasis serait une maladie autoimmune. Le psoriasis touche fréquemment les zones de frottement comme les genoux, les coudes et la région des lombaires ainsi que le cuir chevelu, les mains et les pieds. On distingue plusieurs formes de psoriasis (psoriasis en gouttes, psoriasis pustuleux...), le psoriasis par plaque (ou psoriasis vulgaire) étant la forme la plus courante. L'étiologie du psoriasis demeure à l'heure actuelle mal connue. Un cas sur deux semble d'origine familiale. Différents gènes de prédisposition ont été découverts. Des études récentes suggèrent que le psoriasis résulte également d'anomalies immunitaires. Enfin, le psoriasis peut être déclenché ou aggravé par un certain nombre de facteurs tels que le stress psychologique, le surmenage, l'alcool, le régime alimentaire, la surcharge pondérale, certaines infections ou la prise de médicaments. Les traitements locaux et la photothérapie sont utilisés pour les formes modérées du psoriasis. Les traitements systémiques sont réservés aux formes sévères de psoriasis. Ces traitements présentent une efficacité variable et peuvent induire des effets secondaires gênants.

L'eczéma regroupe des dermatoses inflammatoires érythémato-vésiculeuses prurigineuses. Ces pathologies se développent généralement en plusieurs phases : une phase érythémateuses, une phase vésiculeuse, une phase de suintement et une phase de desquamation. On distingue, entre autres, l'eczéma nummulaire, l'eczéma de contact qui est induit par une réaction allergique vis-à-vis d'un agent exogène, et la dermatite atopique.
La dermatite atopique (également appelée dermite atopique ou eczéma atopique) est une dermatose chronique, évoluant par poussées et qui touche essentiellement les enfants, en particulier les nourrissons. La dermatite atopique est caractérisée par une sécheresse cutanée importante (xérose), par des lésions inflammatoires érythémateuses papuleuses ou vésiculeuses, squameuses, avec éventuellement des fissures et une lichénification des lésions La dermatite atopique touche les convexités des joues, des membres et du cuir chevelu chez les nourrissons. Chez l'enfant plus âgé et l'adulte, les lésions se situent essentiellement au niveau des plis. Tout comme la rosacée et le psoriasis, la pathogénèse de la dermatite atopique est mal connue. Une prédisposition génétique et/ou une anomalie immunitaire est souvent avancée. Certains facteurs peuvent provoquer ou aggraver les poussées de dermatite atopique: régime alimentaire (oeuf, produit laitier, arachide), le stress, l'exposition à certains polluants ou irritants environnementaux (acariens, pollens, eau, calcaire, parfums, métaux...).
Le traitement de la dermatite atopique est essentiellement symptomatique. Il peut comprendre l'application locale de corticoïdes et d'émollient. Des antihistaminiques peuvent être prescrits pour soulager le prurit.

Enfin, le prurit est un désordre fonctionnel qui peut être défini comme « une sensation qui provoque le besoin de se gratter ». Il peut être localisé ou généralisé. Il s'agit d'un symptôme fréquent, en particulier des dermatoses inflammatoires avec lésions cutanées telles que le psoriasis et la dermatite atopique. Néanmoins, certains prurits ne sont pas associés à des lésions cutanées spécifiques (prurit « *sine materia* »). Le prurit peut alors être provoqué par une affection générale, être d'origine neurologique ou psychologique ou être associé à une sécheresse cutanée (xérose). Le prurit peut être causé ou aggravé par une hypersensibilité à des facteurs extérieurs (produits chimiques, variations de température et d'humidité, eau calcaire...). Dans certains cas, le prurit est une manifestation neurologique entretenue et/ou amplifiée par une réaction inflammatoire locale résultant du grattement. Il n'existe pas de traitement général du prurit.

Il existe donc encore à l'heure actuelle un besoin de nouveaux traitements contre les maladies cutanées inflammatoires, en particulier la rosacée.

Les canaux sodium voltage-dépendants sont des acteurs essentiels de l'initiation et de la propagation des potentiels d'actions au niveau des cellules dites « excitables ». Ces canaux sodiques sont principalement exprimés au niveau des neurones du système nerveux central et périphérique et au niveau des cellules musculaires. Il s'agit de protéines transmembranaires comprenant une large sous-unité alpha (a) d'environ 260 kDa associée à une ou plusieurs sous-unités béta de 33 à 38 kDa. La sous-unité alpha forme le coeur du canal et comprend quatre domaines membranaires homologues (I-IV), chacun constitué de 6 segments transmembranaires (S1-S6). Les domaines membranaires de la sous unité alpha sont reliés entre eux par de grandes boucles intracellulaires qui comprennent de nombreux sites de régulation. La sous-unité alpha est responsable des propriétés de conductance et de sélectivité du canal alors que les sous-unités béta sont impliquées dans la stabilisation et dans les propriétés cinétiques du canal. A ce jour, 10 isoformes pour la sous-unité alpha ont été identifiées chez l'homme. Le nom attribué à ces isoformes contient le symbole de l'ion transporté (Na), avec en indice l'élément régulateur (le voltage v). Les chiffres qui suivent désignent la sous-famille de gènes et le numéro associé à l'isoforme considérée. A ce jour, la principale sous-famille de gènes comprend les isoformes Nav1.1 à Nav1.9. Une isoforme supplémentaire, plus éloignée, Nax a été également identifiée. Le pourcentage d'identité de séquence entre les différences isoformes Nav1.1 à Nav 1.9 est d'au moins 45%. Les isoformes Nav1.1 à Nav1.9 se différencient, entre autres, par leur sensibilité à la tétrodotoxine (TTX), un bloqueur très puissant et sélectif des canaux sodiques, et par leur distribution tissulaire. Les isoformes Nav1.1 à Nav1.4, Nav1.6 et Nav1.7 sont dites sensibles à la TTX avec des EC50 de l'ordre du nanomolaire. Les isoformes Nav1.5, Nav1.8 et Nav1.9 sont considérées, quant à elles, comme résistantes à la TTX. A cet égard, l'EC50 de la TTX pour Nav 1.9 est d'environ 200 µM. En ce qui concerne la distribution tissulaire, les isoformes Nav1.1, Nav1.2, Nav1.3 et Nav1.6 sont principalement exprimées au niveau du système nerveux central. L'isoforme Nav1.4 est exprimée principalement au niveau des myocytes squelettiques alors que Nav 1.5 est essentiellement présente, chez l'individu adulte, au niveau des myocytes cardiaques. Enfin, les isoformes Nav 1.7, Nav 1.8 et Nav 1.9 sont exprimées au niveau du système nerveux périphérique (SNP). Nav1.9 a été principalement détecté au niveau des neurones sensitifs des ganglions spinaux où il jouerait un rôle dans la perception de la douleur (nociception), voir Strickland et al., European Journal of Pain, 2008, 12:564-572. Enfin Nax est exprimé au niveau du coeur, de l'utérus, du muscle lisse, des atrocytes et de certains neurones de l'hypothalamus et du système nerveux central. Pour une revue concernant les canaux sodium voltage-dépendants, on pourra se référer à Yu et Caterall, Genome Biology, 2003, 4 :207 ou Caterall et al., Pharmacological Reviews, 2005, 57:397-409.

A la connaissance du Demandeur, aucun lien n'a été établi dans l'état de la technique entre l'expression d'une isoforme de Nav, en particulier Nav1.9, et les maladies cutanées inflammatoires.

### Résumé de l'invention

L'invention concerne une méthode de criblage *in vitro* pour évaluer, cribler ou sélectionner un candidat médicament pour le traitement ou la prévention d'une maladie inflammatoire cutanée, de préférence une rosacée, ladite méthode comprenant :
- l'incubation d'une cellule capable d'exprimer Nav1.9 avec un composé,
- la détermination de l'effet du composé sur l'expression ou l'activité de Nav1.9, et
- la sélection du composé en tant que médicament candidat pour le traitement ou la prévention de la maladie cutanée inflammatoire si le médicament est capable d'inhiber ou de diminuer l'expression ou l'activité de Nav1.9.

La maladie cutanée inflammatoire peut être choisie parmi une rosacée, le psoriasis, le prurit, l'eczéma de contact et la dermatite atopique. De préférence, la maladie cutanée inflammatoire est une rosacée.

Un objet supplémentaire selon l'invention est une méthode *in vitro* de diagnostic d'une maladie cutanée inflammatoire, de préférence une rosacée, chez un patient susceptible d'en être atteint comprenant la détection ou la quantification de l'expression ou de l'activité de Nav 1.9 dans un échantillon biologique du patient d'en être atteint. Dans certains modes de réalisations, la méthode selon l'invention comprend les étapes consistant à :
(a) détecter ou quantifier l'expression ou l'activité de Nav1.9 dans un échantillon biologique du patient, de préférence de peau, et
(b) comparer l'expression ou l'activité de Navl.9 obtenu à l'étape (a) avec l'expression ou l'activité de Nav1.9 détectée ou quantifiée dans un ou plusieurs échantillons biologiques, de préférence de peau, provenant d'un ou plusieurs sujets contrôles, de préférence sains.

La maladie cutanée inflammatoire peut être choisie parmi une rosacée, le psoriasis, le prurit, l'eczéma de contact et la dermatite atopique. De préférence, la maladie cutanée inflammatoire est une rosacée.

Un autre objet selon l'invention est une méthode *in vitro* d'évaluation de l'efficacité d'un traitement thérapeutique d'une maladie cutanée inflammatoire, de préférence une rosacée, chez un patient comprenant les étapes consistant à :
(a) détecter ou quantifier l'expression ou l'activité de Nav1.9 dans un échantillon biologique du patient, de préférence de peau, avant traitement, et
(b) détecter ou quantifier l'activité ou l'expression de Nav1.9 dans un échantillon biologique du patient, de préférence de peau, après traitement, et
(c) comparer l'expression ou l'activité de Navl.9 de l'étape (a) avec celle de l'étape (b).

La maladie cutanée inflammatoire peut être choisie parmi une rosacée, le psoriasis, le prurit, l'eczéma de contact et la dermatite atopique. De préférence, la maladie cutanée inflammatoire est une rosacée.

L'invention a également pour objet une méthode *in vitro* pour suivre la progression d'une maladie cutanée inflammatoire, chez un patient comprenant les étapes consistant à
(a) détecter ou quantifier l'activité ou l'expression de Nav1.9 dans un premier échantillon biologique du patient, de préférence de peau, à un temps t1,
(b) détecter ou quantifier l'expression ou l'activité de Nav1.9 dans un second échantillon biologique, de préférence de peau, à un temps t2 postérieur au temps t1, et
(c) comparer l'expression ou l'activité de Navl.9 de l'étape (b) avec celle de l'étape (a).

La maladie cutanée inflammatoire peut être choisie parmi une rosacée, le psoriasis, le prurit, l'eczéma de contact et la dermatite atopique. De préférence, la maladie cutanée inflammatoire est une rosacée.

Dans des modes de réalisation particuliers, les méthodes selon l'invention ci-dessus décrites sont caractérisées par une ou plusieurs des caractéristiques suivantes :
(i) les échantillons biologiques sont choisis parmi des échantillons de derme, des échantillons d'épiderme et des échantillons de derme et d'épiderme,de préférence des coupes histologiques, et/ou
(ii) la méthode comprend la détection ou la quantification de l'expression de Nav 1.9 dans les échantillons biologiques, et/ou
(iii) l'expression de Nav1.9 est détectée ou quantifiée à l'aide d'un anticorps dirigé contre Nav1.9 dans les échantillons biologiques, de préférence par immunohistochimie.

### Figures

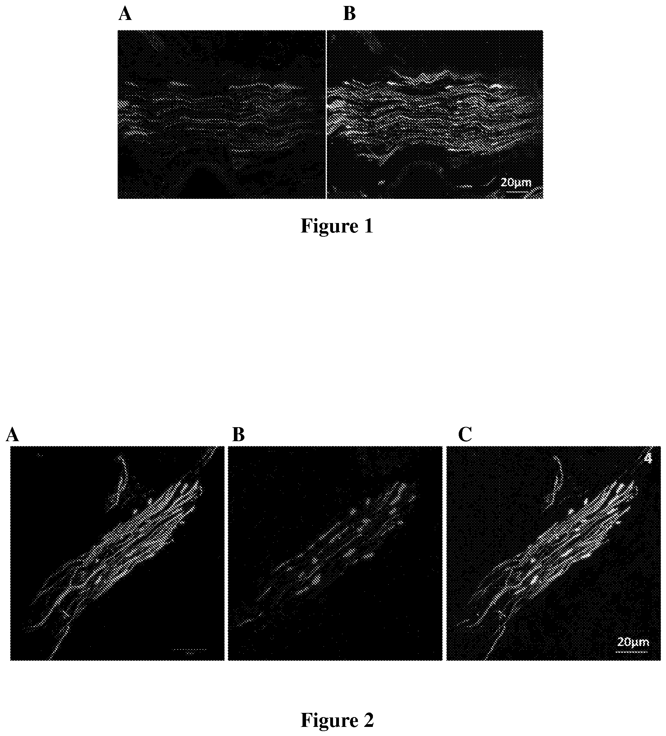

La **Figures 1** montre les résultats d'immunomarquage d'une coupe de derme profond sain avec l'anticorps anti-Navl.9 Rbaa6 (Figure 1A). Le co-marquage avec un anticorps anti-périphérine est illustré à la figure 1B. Une proportion importante proportion des fibres nerveuses du derme marquées à la périphérine sont Nav1.9 positives.
La **Figure 2** montre les résultats d'immunomarquage d'une coupe de derme profond sain avec l'anticorps anti-Navl.9 3881.1 (Figure 2B) et un anticorps anti-périphérine (Figure 2A). Le co-marquage est montré à la figure 2C. Une importante proportion des fibres nerveuses du derme marquées à la périphérine sont également Nav1.9 positives.

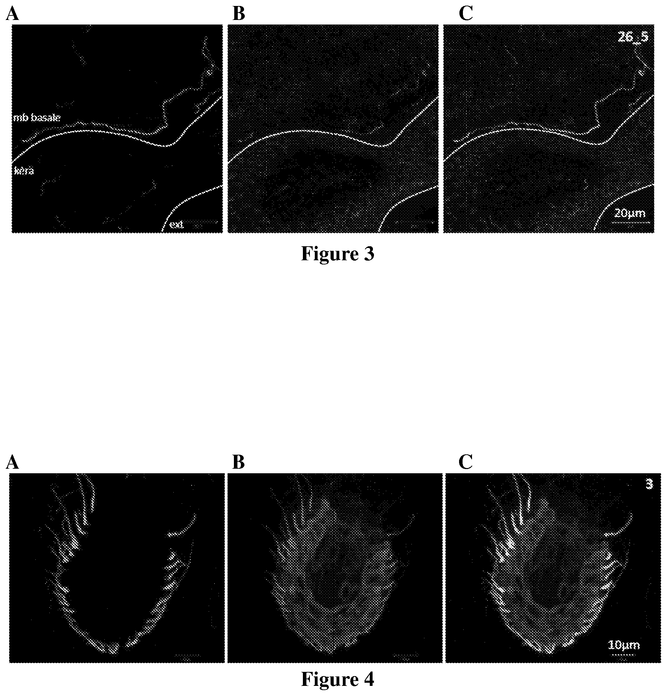

La **Figure 3** montre l'expression localisée de Nav1.9 au niveau d'une fibre nerveuse isolée du derme sain, à proximité de l'interface épiderme-derme. La figure 3A montre le marquage avec l'anticorps anti-périphérine. La figure 3B montre le marquage avec l'anticorps 3881. Le co-marquage de la périphérine et de Nav 1.9 est illustré à la figure 3C. Mb basale : membrane basale, kéra : kératinocyte de l'épiderme, Ext : extérieur.
La **Figure 4** montre l'expression localisée de Nav1.9 au niveau des fibres sensitives innervant le follicule pileux (peau saine). La figure 4A montre le marquage avec l'anticorps anti-périphérine. La figure 4B montre le marquage avec l'anticorps 3881.1. Le co-marquage de la périphérine et de Nav 1.9 est illustré à la figure 4C.

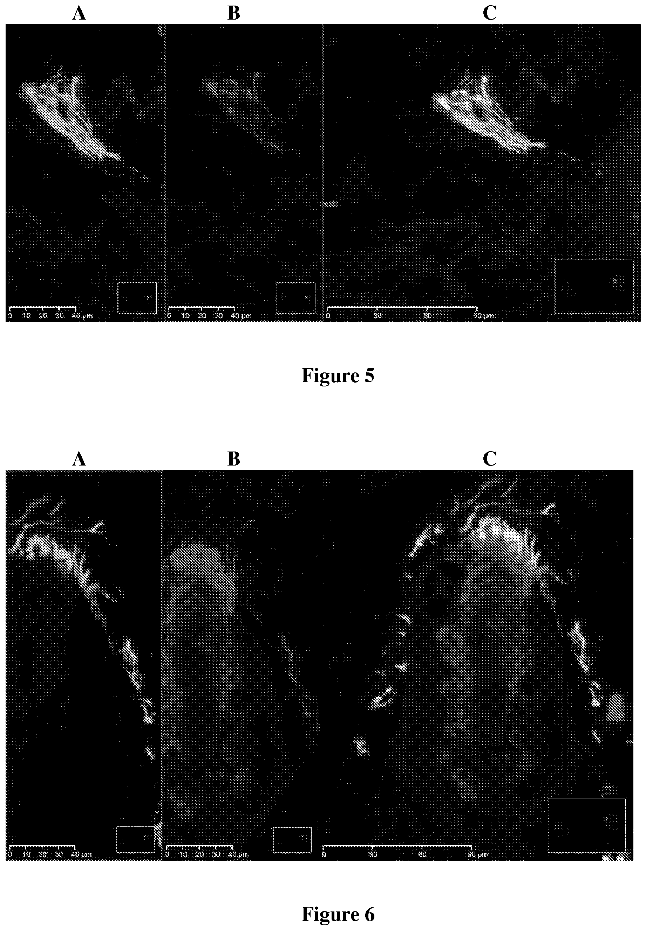

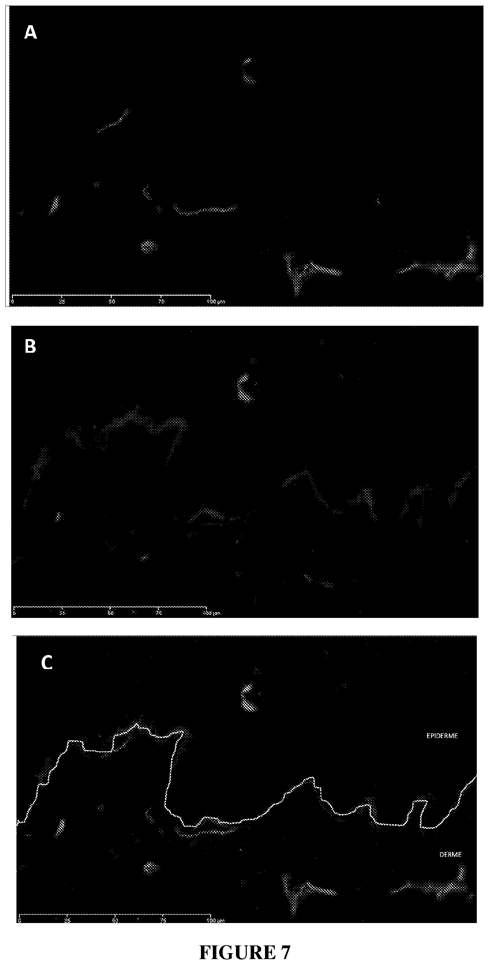

Les **Figures 5, 6** **et** **7** montrent l'immunomarquage d'échantillons cutanés provenant de patients souffrant de rosacée. La Figure 5 correspond à une coupe de derme et la Figure 6 montre un follicule pileux. La Figure 7 montre l'immunomarquage d'une coupe cutanée de « derme - épiderme ». Figures 5A, 6A et 7A: marquage avec l'anticorps anti-périphérine. Figures 5B, 6B et 7B : marquage avec l'anticorps 3881.1. Le co-marquage est montré dans les Figures 5C, 6C et 7C. Nav1.9 est détecté avec l'anticorps 3881.1 au niveau des fibres nerveuses sensitives dans le derme, au niveau du follicule pileux et à l'interface du derme et de l'épiderme dans la peau de patients atteints de rosacée.

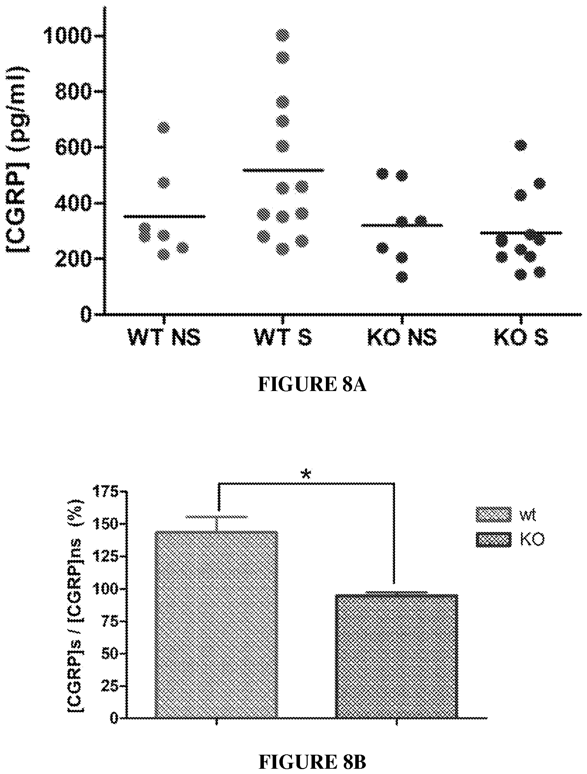

Les **Figure 8A et 8B** montrent les résultats des expériences de libération de CGRP (calcitonin gene-related peptide) chez des cultures primaires de neurones, issus de ganglions spinaux de souris sauvages (WT) ou invalidées pour le gène de Nav1.9 (KO), après traitement (S) à la capsaïcine (15 min, 300 nM) ou sans traitement à la capsaïcine (NS) (voir Example 3 ci-après). La **Figure 8A** montre les concentrations de CGRP (pg/ml) détectées dans le surnageant pour chaque culture cellulaire testée ainsi que les concentrations moyennes de CGRP pour chaque groupe d'expériences (barre noire). La **Figure 8B** montre les rapports (en %) de la concentration moyenne de CGRP dans le surnageant, après stimulation, sur la concentration moyenne, avant stimulation, pour les cultures cellulaires provenant de souris WT et KO. On observe une augmentation d'environ 43,5% de la concentration de CGRP après traitement à la capsaïcine pour les cultures cellulaires obtenues à partir des souris WT. En revanche, aucune augmentation n'est observée pour les cultures neuronales provenant de souris KO après stimulation à la capsaïcine.

### Description détaillée de l'invention

Le Demandeur a montré par des expériences d'immunomarquage que le canal sodique Nav1.9 est exprimé par les fibres sensitives de la peau (Exemple 1). Une forte expression du canal sodique Nav 1.9 a été détectée au niveau des fibres nerveuses sensitives d'échantillons de peau provenant de patients atteints par la rosacée (Exemple 2). Le Demandeur a, par ailleurs, montré que Nav1.9 contrôle le relargage du peptide CGPR (calcitonin gene-related peptide), un médiateur de l'inflammation par les neurones sensitifs suite à une stimulation par la caspaïcine. Ces résultats suggèrent très fortement une implication de Nav1.9 dans la potentialisation de la réponse inflammatoire consécutive à une stimulation des fibres sensitives, en particulier au niveau de la peau. Le Demandeur a donc montré, pour la première fois, que Nav1.9 est une cible thérapeutique de choix pour le traitement des maladies cutanées inflammatoires, en particulier la rosacée.

### ▪ Méthode de criblage de composés pour le traitement d'une maladie cutanée inflammatoire

Un objet de la présente invention est une méthode de criblage *in vitro* pour évaluer, cribler ou sélectionner un médicament candidat pour le traitement ou la prévention d'une maladie inflammatoire cutanée comprenant les étapes consistant à :
- incuber une cellule capable d'exprimer Nav1.9 avec un composé,
- déterminer l'effet du composé sur l'expression ou l'activité de Nav1.9, et
- sélectionner le composé en tant que médicament candidat si le composé est capable d'inhiber l'expression ou l'activité de Navl.9.

Au sens de l'invention, « une cellule » désigne une cellule isolée ou un ensemble de cellules tel qu'une culture cellulaire. La cellule utilisée peut être toute cellule exprimant de manière endogène ou de manière recombinante Nav 1.9. A titre d'exemple, la cellule peut être un neurone ou une culture de neurones primaire, par exemple une culture de neurones spinaux. Il peut s'agir également d'une cellule hôte telle que Cos-7, CHO, BHK, et HEK-293, exprimant de manière stable ou transitoire Nav 1.9.

La maladie cutanée inflammatoire peut être choisie parmi une rosacée, le psoriasis, le prurit, et un eczéma tel que la dermatite atopique. De préférence, la maladie cutanée inflammatoire est une rosacée. Dans certains modes de réalisations, la maladie cutanée inflammatoire est choisie parmi la rosacée érythématotélangiectasique (sous-type 1), la rosacée papulopustulaire (sous-type 2), la rosacée phymateuse (sous-type 3), la rosacée oculaire (sous-type 4) et une variante granulomateuse de la rosacée.

L'effet du composé sur l'expression de Nav 1.9 peut être déterminé en quantifiant la protéine Nav 1.9 ou l'ARN messager codant pour Nav 1.9. L'homme du métier peut ainsi utiliser des méthodes bien connues pour quantifier l'ARNm, par exemple, la RT-PCR quantitative, le Northern blot ou encore les techniques basées sur l'hybridation de l'acide nucléique cible (ARNmessager ou ADNc) avec une sonde oligonucléotide. A titre alternatif, on peut également utiliser un système recombinant comprenant un gène rapporteur, par exemple le gène de la luciférase ou de la GFP, dont l'expression est mise sous le contrôle du promoteur du gène Nav 1.9. L'effet du composé pourra être alors déterminé en détectant l'expression du gène rapporteur. A titre alternatif, l'effet du composé peut être évalué en quantifiant la protéine Nav 1.9. A cette fin, on peut utiliser des techniques d'immuno-dosage, par exemple un test ELISA ou un dosage radio-immunologique (RIA), l'immunohistochimie ou encore la spectrométrie de masse, éventuellement couplé à la chromatographie en phase liquide (LC-MS).

L'effet du composé sur l'activité de Nav 1.9, c'est-à-dire son éventuelle activité inhibitrice, peut être déterminée par des méthodes bien connues de l'homme du métier. Il peut s'agir d'un test de liaison au canal Nav 1.9, d'un test d'électrophysiologie comme un patch clamp, un test d'influx d'ions radioactifs (radiotraceur), par exemple ²²Na⁺, ou d'un test basé sur l'utilisation de sondes fluorescentes sensibles au voltage ou sur le transfert d'énergies entre molécules fluorescentes (FRET). L'homme du métier peut ainsi s'inspirer des tests d'activité décrits dans Castle et al., 2009, Combinatorial Chemistry & High Throughput Screening, 12, 107-122 et Zheng et Laszlo, 2004, ASSAY and Drug Development Technologies, 2(5): 543-552 pour la mise en oeuvre pratique du procédé de criblage selon l'invention, le contenu de ces deux publications étant incorporé ici par référence. L'homme du métier pourra également s'inspirer des travaux de Bosmans et al (J Gen Physiol, 2011, 138(1) :59-72) qui décrit l'obtention de chimères Nav1.9-Kv1.2 et leurs mises en oeuvre dans un test d'activité.

On considère que le composé inhibe l'expression ou l'activité Nav 1.9 si le taux d'expression ou l'activité de Nav 1.9 dans la cellule incubée avec le composé est au plus égale à 90%, de préférence au plus égale à 80% du taux d'expression ou de l'activité de Nav 1.9 détectée dans une cellule témoin qui n'a pas été incubée avec le composé. Une expression ou une activité au plus égale à 90% englobe une expression ou une activité au plus égale à 80%, 70%, 60%, 50%, 40%, 30% 20%, 10%, 5% de l'expression ou de l'activité de Nav 1.9 dans la cellule témoin. Dans un mode de réalisation particulier, la méthode de criblage selon l'invention peut comprendre une étape consistant à tester le médicament candidat sélectionné sur un modèle de maladie inflammatoire cutanée, de préférence un modèle in vitro. Le modèle est de préférence un modèle de rosacée.

Les composés testés dans la méthode de criblage selon l'invention peuvent être des composés naturels ou synthétiques provenant de chimiothèques ou de banques de composés. Il peut s'agir également de composés pré-selectionnés, par exemple, par criblage *in silico,* la structure du canal sodique Nav 1.9 étant connue.

### ▪ Méthode de diagnostic in vitro

La présente invention concerne également une méthode *in vitro* de diagnostic d'une maladie cutanée inflammatoire chez un patient susceptible d'en être atteint, ladite méthode comprenant la détection et/ou la quantification de l'activité et/ou l'expression de Nav 1.9 dans un échantillon biologique provenant du patient. Dans certains modes de réalisation, cette méthode comprend les étapes consistant à :
(a) détecter ou quantifier l'expression ou l'activité de Nav1.9 dans un échantillon biologique du patient, de préférence de peau,
(b) comparer l'expression ou l'activité de Nav1.9 obtenue à l'étape (a) avec l'expression ou l'activité de Nav1.9 détectée ou quantifiée dans un ou plusieurs échantillons biologiques, de préférence de peau, provenant d'un ou plusieurs sujets contrôles.
Dans certains modes de réalisations, le ou les sujets contrôles sont des individus souffrant de la maladie cutanée inflammatoire que l'on souhaite diagnostiquer chez le patient. Dans ce mode de réalisation, on peut considérer que le patient souffre de la maladie cutanée inflammatoire si l'expression ou l'activité de Nav 1.9 déterminée à l'étape (a) est au moins égale à celle des sujets contrôles. Si l'expression ou l'activité de Nav 1.9 déterminée à l'étape (a) est significativement inférieure à celle détectée pour les sujets contrôles, le diagnostic est négatif. Enfin, si l'expression ou l'activité de Nav 1.9 déterminée à l'étape (a) n'est que très légèrement inférieure à celle déterminée pour les sujets contrôles, le patient peut être diagnostiqué comme un patient à risque, c'est-à-dire un patient susceptible de développer la maladie cutanée inflammatoire.
Dans d'autres modes de réalisation, le ou les sujets contrôles correspondent à des patients sains, c'est-à-dire à des individus qui ne souffrent pas de la maladie cutanée inflammatoire. Dans ces modes de réalisations, le patient est diagnostiqué comme souffrant de la maladie cutanée inflammatoire, ou susceptible d'en souffrir, si son échantillon biologique présente une expression ou une activité pour Nav 1.9 supérieure à celle du ou des échantillons provenant des patients sains. En revanche, si l'activité ou l'expression de Nav 1.9 déterminée à l'étape (a) est inférieure ou égale à celle des échantillons provenant des patients contrôles (sains), alors le diagnostic peut être considéré comme négatif. On peut considérer que l'échantillon du patient est positif si l'échantillon présente une expression ou une activité pour Nav 1.9 au moins 10% supérieure, de préférence au moins 20% supérieure à celui du ou des échantillons des patients sains.
La détection ou la quantification du ou des échantillons biologiques du ou des sujets contrôles peuvent être concomitantes à celles effectuées pour l'échantillon du patient ou provenir de données collectées antérieurement et disponibles, par exemple, sur une base de données.

Un objet supplémentaire selon l'invention est une méthode *in vitro* d'évaluation de l'efficacité d'un traitement thérapeutique d'une maladie cutanée inflammatoire, chez un patient comprenant les étapes consistant à :
(a) détecter ou quantifier l'expression ou l'activité de Nav1.9 dans un échantillon biologique du patient avant traitement,
(b) détecter ou quantifier l'expression ou l'activité de Nav1.9 dans un échantillon biologique du patient après traitement,
(c) comparer l'expression ou l'activité de Nav1.9 de l'étape (a) avec celle déterminée à l'étape (b).
L'efficacité du traitement est déterminée en comparant les expressions de Nav 1.9 dans les échantillons (a) et (b). Si l'expression ou l'activité de Nav 1.9 est plus faible dans l'échantillon de l'étape (b) que dans l'échantillon de l'étape (a), ceci signifie que le traitement est efficace. S'il y a une augmentation de l'expression de Nav 1.9, on peut considérer que le traitement est peu actif ou inactif selon l'importance de la variation observée. Dans certains modes de réalisation, on considère que le traitement a un effet thérapeutique sur la maladie cutanée inflammatoire s'il implique une diminution d'expression ou d'activité de Nav 1.9 d'au moins 10%, de préférence d'au moins 20%. Les échantillons biologiques, prélevés avant traitement et après traitement, sont de préférence des échantillons de peau.

La présente invention a également pour objet une méthode *in vitro* pour suivre la progression d'une maladie cutanée inflammatoire chez un patient comprenant les étapes consistant à :
(a) détecter ou quantifier l'activité ou l'expression de Nav1.9 dans un premier échantillon biologique, de préférence de peau, prélevé chez le patient à un temps t1,
(b) détecter ou quantifier l'activité ou l'expression de Nav1.9 dans un second échantillon biologique, de préférence de peau, prélevé chez le patient, le second échantillon ayant était prélevé à un temps t2 postérieur au temps t1,
(c) comparer l'expression ou l'activité de Nav1.9 de l'étape (b) avec celle déterminée à l'étape (a).
La comparaison de l'expression ou de l'activité de Nav 1.9 dans l'étape (c) est un critère permettant de déterminer la progression ou le stade de la maladie cutanée inflammatoire. Il est ainsi possible de déterminer si la maladie est en régression, stationnaire ou s'aggrave.
Si l'échantillon de l'étape (b) présente un taux d'expression de Nav 1.9 inférieur à l'expression ou à l'activité déterminée à l'étape (a), il peut être conclu que la maladie cutanée inflammatoire est en régression. A l'inverse, si l'échantillon de l'étape (b) présente une expression ou une activité pour Nav 1.9 supérieure à celle mesurée à la première étape, la maladie cutanée inflammatoire est en voie de progression.
Dans le cas de maladies chroniques telles que la rosacée, la méthode selon l'invention permet également d'anticiper la survenue d'une nouvelle crise ou poussée et de mettre en place un traitement prophylactique.
Dans certains modes de réalisation, le temps t2 est postérieur d'au moins 1 mois, de préférence d'au moins 6 mois, voire d'au moins 1 an au temps t1.
L'invention a également pour objet des méthodes pour générer des données et/ou des informations utiles pour diagnostiquer, et/ou pour prédire et/ou pour suivre l'évolution d'une maladie cutanée inflammatoire, chez un patient, ces méthodes pouvant contenir l'une quelconque des combinaisons d'étapes ci-dessus décrites. L'invention a en outre pour objet une méthode pour générer des données et/ou des informations utiles pour déterminer l'efficacité d'un traitement chez un patient souffrant d'une maladie cutanée inflammatoire.
Dans l'ensemble des méthodes selon l'invention décrites ci-avant, la maladie cutanée inflammatoire peut être choisie parmi une rosacée, le psoriasis, le prurit, l'eczéma de contact, et la dermatite atopique.
La maladie cutanée inflammatoire préférée est une rosacée, par exemple la rosacée érythématotélangiectasique (sous-type 1), la rosacée papulopustulaire (sous-type 2) ou encore une variante granulomateuse de la rosacée.
Pour la mise en oeuvre de l'une quelconque des méthodes ci-dessus décrites, le ou les échantillons biologiques sont de préférence un ou des échantillons de peau. Il peut s'agir d'échantillons de derme, d'épiderme ou de derme et d'épiderme. Dans certains modes de réalisation, il s'agit d'une biopsie de peau.

De préférence, les méthodes selon l'invention comprennent la détection ou la quantification de l'expression de Nav 1.9 dans le ou les échantillons biologiques.
Dans les méthodes selon l'invention, l'expression de Nav 1.9 peut être détectée ou quantifiée selon des méthodes bien connues de l'homme du métier.
A cette fin, l'homme du métier peut rechercher à quantifier l'ARN messager codant pour Nav 1.9, par exemple par RT-PCR quantitative et/ou par des techniques d'hybridation, par exemple à l'aide d'une sonde marquée ou par l'utilisation d'une puce ADN. Dans d'autres modes de réalisation, les méthodes selon l'invention comprennent la quantification de la protéine Nav 1.9 dans les échantillons biologiques, par exemple, par western blot, par spectrométrie de masse, par immuno-dosage, en particulier par ELISA, par immuno-histochimie, par exemple par immunomarquage par fluorescence. De préférence, l'expression de Nav 1.9 est détectée ou quantifiée par détection ou quantification de la protéine Nav1.9 en tant que telle.
L'activité de Nav 1.9 peut être également déterminée ou quantifiée selon des méthodes bien connues de l'homme du métier. On préférera la mise en oeuvre d'une technique d'électrophysiologie, type patch clamp, effectuée sur des cellules isolées de l'échantillon biologiques ou encore, ou d'une méthode indirecte basée sur le dosage de neuropeptides pro-inflammatoires ou l'évaluation de la réponse inflammatoire (oedème, rougeur) au niveau de la peau.
L'échantillon biologique peut subir un certain nombre de traitements pour permettre la quantification ou la détection de l'activité ou de l'expression de Nav 1.9.
A titre d'exemple, pour détecter ou quantifier Nav 1.9 par immunohistochimie, l'échantillon biologique peut être une coupe histologique de derme ou de derme-épiderme qui est, au préalable, soit cryofixée, soit fixée chimiquement et éventuellement incluse dans une résine. A titre d'exemple supplémentaire, l'échantillon biologique, de préférence de derme, peut être broyé, centrifugé puis extrait afin d'obtenir un extrait protéique issu des membranes cellulaires adapté à la mise en oeuvre d'un western blot.

Dans des modes de réalisation particuliers, les méthodes selon l'invention sont caractérisées par une ou plusieurs des caractéristiques (voire toutes les caractéristiques) suivantes :
(i) les échantillons biologiques sont des échantillons de peau, par exemple de derme, d'épiderme et d'épiderme-derme. De préférence, il s'agit de coupes histologiques, et/ou
(ii) la méthode comprend la détection ou la quantification de l'expression de Nav 1.9 dans les échantillons biologiques, et/ou
(iii) l'expression de Nav 1.9 est détectée ou quantifiée à l'aide d'un anticorps dirigé contre Nav 1.9 dans les échantillons biologiques, de préférence par immunohistochimie.
L'anticorps peut être un anticorps polyclonal ou monoclonal. De préférence, l'anticorps est dirigé contre un ou plusieurs épitopes inclus dans l'extrémité N-terminale, dans la boucle intracellulaire liant les domaines membranaires I et II ou dans la boucle intracellulaire liant les domaines membranaires II et III de Nav 1.9 humain. L'anticorps peut être couplé à un moyen de détection tel qu'une molécule fluorescente, un chromophore ou une enzyme comme la peroxydase de raifort (HRP), la phosphatase alcaline, la 3-galactosidase, ou la glucose-6-phosphate déshydrogénase. A titre alternatif, la détection peut être effectuée à l'aide d'un anticorps secondaire.

Les méthodes selon l'invention peuvent aussi comprendre la détection d'un marqueur supplémentaire ou l'évaluation d'un symptôme particulier de la maladie inflammatoire cutanée. A titre d'exemple, lorsque la maladie inflammatoire cutanée est la rosacée, les méthodes selon l'invention peuvent comprendre la détection d'un marqueur supplémentaire ou d'un symptôme de la rosacée tel que les papules, l'hyperréactivité vasculaire, l'érythème, ou les sensations de brûlures, picotements ou cisaillements.

Enfin, l'invention a également pour objet l'utilisation d'un anticorps dirigé contre Nav 1.9 pour le diagnostic, le pronostic et/ou la prédiction *in vitro* d'une maladie cutanée inflammatoire, ou encore l'utilisation dudit anticorps pour le suivi *in vitro* de l'évolution d'une maladie cutanée inflammatoire ou pour évaluer *in vitro* l'efficacité d'un médicament pour le traitement d'une maladie cutanée inflammatoire, la maladie cutanée inflammatoire peut être choisie parmi une rosacée, le psoriasis, le prurit, l'eczéma de contact, et la dermatite atopique. La maladie cutanée inflammatoire étant de préférence la rosacée.

D'autres aspects et avantages de la présente invention apparaîtront à la lecture des exemples qui suivent. Ces exemples doivent être considérés comme illustratifs et en aucun cas comme limitant la portée de l'invention.

### Exemples

### Exemple 1 : Détection de l'expression de Nav1.9 par les fibres nerveuses sensitives cutanées

La détection de l'expression de Nav1.9 au niveau des fibres nerveuses cutanées a été effectuée par immunohistofluorescence à l'aide d'anticorps polyclonaux dirigés contre la sous-unité alpha du canal sodium voltage-dépendant Navl.9 humain. L'anticorps Rbαa6 est dirigé contre la boucle intracellulaire liant les domaines transmembranaires II et III de Nav1.9 humain. L'anticorps 3881.1 est dirigé contre la boucle intracellulaire I-II de Nav1.9.

Il a été montré que ces anticorps polyclonaux sont spécifiques de Nav1.9 humain. En particulier, ils ne se lient pas aux canaux sodiques Nav 1.5 et Nav 1.7.

Un co-marquage à l'aide d'un anticorps dirigé contre la périphérine, une protéine spécifique du tissu nerveux périphérique, a été réalisé afin de localiser les fibres nerveuses.

De le peau entière a été utilisée et les différents compartiments ont été identifiés et analysés : derme profond, derme, interface derme-épiderme et follicule pileux.

### - Prélèvement et préparation des échantillons

Les échantillons de peau ont été préparés de la manière suivante :
Après désinfection de la peau, des biopsies de peau de 3 mm ont été réalisées chez un patient sain soumis à une anesthésie locale au site de la biopsie. La biopsie est ensuite lavé dans du PBS pH 7.2 et placé dans un tube eppendorf dans de azote liquide. L'eppendorf est ensuite stocké à -80°C jusqu'à inclusion en matrice OCT. Les coupes de congélation de 10 µm d'épaisseur sont ensuite réalisées.

### - Protocole de marquage par immunofluorescence

Les coupes sont incubées 1h30 à température ambiante dans un milieu de saturation : PBS + 3% (masse/volume) BSA + 0.3% (volume/volume) Triton X-100. Les anticorps primaires (anti-Nav1.9 ou anti-périphérine) sont ajoutés au milieu de saturation pour une incubation de 12 h à 4°C en chambre humide. Les coupes sont ensuite rincées 4 fois 15 min dans du PBS avant 45 min d'incubation à à température ambiante avec les anticorps secondaires dans du PBS + 3% (masse/volume) BSA en chambre humide. Enfin, les coupes sont rincées 4 fois 15 min dans du PBS avant d'être montées dans un milieu de montage (Mowiol ou Prolong Gold).

### - Résultats

Les Figures 1 et 2 montrent le marquage par immunofluorescence de coupe de dermes profond par l'anticorps anti-périphérine (Figures 2A), l'anticorps Rbaa6 (Figure 1B), et l'anticorps 3881.1 (Figure 2B). Les résultats d'immunomarquage obtenus par les anticorps Rbαa6 et 3881.1 sont similaires. On observe une expression du canal sodique Nav1.9 localisée au niveau des fibres sensitives du derme profond. Des zones de co-localisation de la périphérine et de Nav1.9 sont mises en évidence dans les Figures 1C et 2C.

Les Figures 3 et 4 montrent les résultats d'immuno-marquage de la périphérine et de Nav1.9 au niveau de l'interface épiderme-derme et d'un follicule pileux respectivement. Ces clichés d'immuno-marquage ont été obtenus avec l'anticorps 3881.1

La Figure 3 montre que l'expression localisée de Nav1.9 au niveau d'une fibre nerveuse isolée du derme. Un résultat analogue est observé au niveau du follicule pileux où Nav1.9 est exprimée, de manière localisée, par les fibres sensitives innervant le follicule. Un léger marquage non-spécifique est néanmoins visible des kératinocytes du follicule. Aucun marquage significatif n'a été détecté dans l'épiderme.

### - Conclusions

Ces expériences d'immuno-marquage montrent que le canal sodique Nav1.9 est exprimé de manière basale au niveau des fibres sensitives cutanées, en particulier du derme.

### Exemple 2 : Surexpression de Nav1.9 par les fibres sensitives cutanées chez des patients atteints de rosacée.

### Préparation des échantillons

Les échantillons de peau ont été préparés de la manière suivante:
Après désinfection de la peau, des biopsies de peau de 3 mm ont été réalisées chez un patient atteint de Rosacée soumis à une anesthésie locale au site de la biopsie. La biopsie est ensuite lavé dans du PBS pH7.2 et placé dans un tube eppendof dans de azote liquide. L'eppendorf est ensuite stocké à -80°C jusqu'à inclusion en matrice OCT. Les coupes de congélation de 10 µm d'épaisseur sont ensuite réalisées.

### Protocole de marquage par immunofluorescence

Le protocole d'immunomarquage est similaire à celui décrit pour l'Exemple 1

Les anticorps suivants ont été utilisés :
▪ Pour la périphérine, l'anticorps primaire est un anticorps monoclonal (Millipore, MAB1527) de souris et l'anticorps secondaire est un anticorps anti-souris d'âne couplé à l'Alexa488 (Invitrogen, A-21202).
▪ Pour Nav1.9, l'anticorps primaire est l'anticorps polyclonal 3881.1 de lapin et l'anticorps secondaire est un anticorps anti-lapin d'âne couplé à TRITC (Jackson Immunoresearch).

### Résultats

Les Figures 5, 6 et 7 montrent les résultats d'immuno-marquage de la périphérine et de Nav1.9 au niveau du derme et d'un follicule pileux provenant d'échantillons de peau atteints par la rosacée. Nav1.9 est localisé au niveau des fibres sensitives du derme et du follicule pileux. Alors qu'un marquage relativement homogène a été observé pour les échantillons de peau saine, on observe la présence de clusters d'expression c'est-à-dire de zones localisées (spots) présentant une expression très élevée de Nav 1.9. La Figure 7 montre par ailleurs une expression de Nav 1.9 dans l'épiderme, ce qui n'avait pas été observé pour les échantillons sains.

Ces résultats préliminaires montrent donc une surexpression de Nav 1.9 au niveau d'échantillons de peau atteints par la rosacée, dans le derme et l'épiderme.

### Exemple 3 : Mise en évidence de l'implication du canal sodique Nav1.9 dans la réponse neuro-inflammatoire.

L'objectif de cette étude était de mettre en évidence l'impact de Nav1.9 sur la libération du peptide relié au gène calcitonine (ou calcitonin gene-related peptide (CGRP)). Le peptide CGRP est un puissant vasodilatateur et un médiateur de la douleur au niveau système nerveux périphérique et central. CGRP est également impliqué dans l'inflammation neurogénique périphérique, en particulier de la peau. A cette fin, le Demandeur a comparé la libération de CGRP par des cultures primaires de neurones, après ou sans traitement à la capsaïcine. Les cultures primaires de neurones ont été obtenues à partir de ganglions spinaux (Dorsal Root Ganglion-DRG) prélevés chez des souris sauvages (wt) et des souris chez lesquelles le gène du canal sodique Nav1.9 a été invalidé (souris KO pour Nav1.9).

### - Cultures primaires de neurones provenant de ganglions spinaux de souris adultes.

Les prélèvements de ganglions spinaux ont été réalisés chez des souris C57BL6 mâles âgées de 2 à 3 mois. En moyenne, 20 ganglions spinaux ont été prélevés par souris, ce qui correspond à un total de 50 000 et 80 000 neurones/souris.

Les neurones collectés ont été ensemencés dans des plaques 96 puits à raison de 10 000 cellules par puits. Les puits ont été préalablement recouverts de poly-L-Lysine (12µg/cm²) et laminine (6µg/cm²) pour favoriser l'adhésion des neurones. Les cellules ont été incubées 24h à 37°C avant le début du test dans un milieu de culture adapté (D-MEM supplémenté en glucose, NEAA,L-glutamine, pyruvate, NGF (Nerve Growth Factor 2.5s mouse submaxillary) GDNF (Glial cell-line Derived Neurotrophic Factor) et sérum de veau foetal).

Au total, les cultures cellulaires ont été obtenues à partir de 12 souris (6 sauvages et 6 KO). 5 à 8 puits de culture primaire de neurones ont été préparés par souris.

### - Quantification de la libération de CRGP dans le milieu après traitement à la capsaïcine

Une partie des cultures primaires (3 à 5 puits de culture par souris) a été incubées 15 min en présence de capsaïcine à 300 nM. La seconde partie des cultures primaires (2 à 3 puits de culture par souris) n'a subi aucun traitement.

Le dosage de CRGP libéré dans le surnageant a été réalisé par dosage ELISA en utilisant le Kit SPIbio ref. A05482 destiné au dosage du CRGP de rat, mais qui est utilisable également pour quantifier le CRGP de souris.

Brièvement, les surnageants des cultures primaires ont été prélevés et dilués au 1/5 avant d'être rajoutés dans les puits d'une microplaque recouverts par un anticorps monoclonal anti-CGRP. Un second anticorps anti-CRPG (polyclonal), couplé à l'acétylcholine estérase, a été ensuite rajouté. Après incubation et rinçage, la détection a été réalisée à l'aide du réactif d'Ellman (DTNB) par mesure de l'absorbance à 405 ou 414 nm.

### - Résultats

Les résultats sont illustrés par les Figure 8A et 8B.

Pour les cultures primaires obtenues à partir de souris sauvages (WT), on observe que le traitement à la capsaïcine a entraîné une augmentation significative de la quantité de CGRP (+43,5%) présente dans le surnageant. En revanche, aucune variation de la quantité de CGRP libéré dans le surnageant n'a été observée pour les cultures primaires provenant de souris KO. Avec ou sans traitement à la capsaïcine, la quantité de CGRP détecté dans le surnageant est équivalente au niveau basal de CGRP du surnageant des cellules WT (avant stimulation capsaïcine).

Nav1.9 est donc impliqué dans la régulation de la libération de CGRP consécutivement à une stimulation à la capsaïcine.

### Exemple 4 : Quantification du pool intracellulaire de CGRP chez des cultures neuronales obtenues à partir de souris sauvages (WT) et de souris KO pour Nav1.9 (KO).

### - Cultures primaires de neurones provenant de ganglions spinaux de souris adultes.

Le protocole utilisé est identique à celui de l'Exemple 3. Les cultures neuronales primaires ont été préparées à partir de 4 souris KO et 4 souris WT.

### - Dosage du pool intracellulaire de CGRP.

Après incubation 24h à 37°C et rinçage, les cultures primaires ont été lysées au Triton X100 à 1%. Le dosage du peptide CGRP dans les lysats cellulaires a été réalisé par le test Elisa décrit dans l'Exemple 3.

Il s'avère que la concentration moyenne de CRPG pour les cultures primaires provenant de souris KO est légèrement inférieure à celle obtenue pour les cultures primaires provenant des souris sauvage. Néanmoins, la différence observée n'est pas significative.

En conséquence, la différence de relargage de CGRP, sous stimulation capsaïcine, observée pour les cultures neuronales primaires WT et KO, dans l'exemple 3, n'est pas attribuable à un déficit intracellulaire de CGRP chez les cellules neuronales primaires KO.

### Conclusion

Les résultats de libération du peptide CGRP par les cultures neuronales primaires, provenant de souris wt et KO, après ou sans stimulation à la capsaïcine, et les résultats d'immunomarquage de coupes de derme suggèrent très fortement l'implication de Nav1.9 dans la potentialisation de l'inflammation neurogène observée au niveau de peau dans les pathologies cutanées inflammatoires. Nav1.9 constitue donc une cible thérapeutique d'intérêt pour le traitement des maladies cutanées inflammatoires, en particulier la rosacée.

## Revendications

1. Méthode de criblage *in vitro* pour évaluer, cribler ou sélectionner un composé destiné au traitement ou à la prévention d'une maladie inflammatoire cutanée, ledit procédé comprenant :
- l'incubation d'une cellule capable d'exprimer Navl.9 avec le composé,
- la détermination de l'effet du composé sur l'expression ou l'activité de Nav1.9, et
- la sélection du composé s'il est capable d'inhiber l'expression ou l'activité de Nav1.9.

2. Méthode selon la revendication 1, dans laquelle la maladie cutanée inflammatoire est la rosacée.

3. Méthode *in vitro* de diagnostic d'une maladie cutanée inflammatoire chez un patient susceptible d'en être atteint, comprenant la détection et/ou la quantification de l'expression et/ou de l'activité de Nav 1.9 dans un échantillon biologique du patient.

4. Méthode selon la revendication 3, dans laquelle la maladie cutanée inflammatoire est la rosacée.

5. Méthode *in vitro* selon la revendication 3 ou 4, comprenant les étapes consistant à :
(a) détecter ou quantifier l'expression ou l'activité de Nav1.9 dans un échantillon biologique, de préférence de peau, du patient, et
(b) comparer l'expression ou l'activité de Navl.9 obtenu à l'étape (a) avec l'expression ou l'activité de Nav1.9 détectée ou quantifiée dans un ou plusieurs échantillons biologiques, de préférence de peau, provenant d'un ou plusieurs sujets contrôles, de préférence sains.

6. Méthode *in vitro* d'évaluation de l'efficacité d'un traitement thérapeutique d'une maladie cutanée inflammatoire chez un patient comprenant les étapes consistant à :
(a) détecter ou quantifier l'expression ou l'activité de Nav1.9 dans un échantillon biologique du patient, de préférence de peau, avant traitement,
(b) détecter ou quantifier l'expression ou l'activité de Nav1.9 dans un échantillon biologique du patient, de préférence de peau, après traitement, et
(c) comparer l'expression ou l'activité de Nav1.9 de l'étape (a) avec celle déterminée à l'étape (b).

7. Méthode *in vitro* pour suivre la progression d'une maladie cutanée inflammatoire chez un patient comprenant les étapes consistant à
(a) détecter ou quantifier l'expression ou l'activité de Nav1.9 dans un premier échantillon biologique du patient, de préférence de peau, à un temps t1,
(b) détecter ou quantifier l'expression ou l'activité de Nav1.9 dans un second échantillon biologique du patient, de préférence de peau, à un temps t2 postérieur au temps t1, et
(c) comparer l'expression ou l'activité de Navl.9 de l'étape (b) avec celle de l'étape (a).

8. Méthode selon l'une quelconque des revendications 3 à 7 comprenant une ou plusieurs des caractéristiques suivantes :
(i) l'échantillon biologique est un échantillon de derme, un échantillon de derme-épiderme ou un échantillon d'épiderme, de préférence une coupe histologique, et/ou
(ii) la méthode comprend la détection ou la quantification de l'expression de Nav 1.9 dans l'échantillon biologique, et/ou
(iii) l'expression de Nav 1.9 est détectée et/ou quantifiée à l'aide d'un anticorps dirigé contre Nav 1.9, de préférence par immunohistochimie, et/ou
(iv)la maladie cutanée inflammatoire est choisie dans le groupe constitué par la rosacée, le psoriasis, le prurit, l'eczéma et la dermatite atopique, de préférence la rosacée.

9. Méthode selon l'une des revendications 6 à 8, dans laquelle la maladie cutanée inflammatoire est la rosacée.

## Patentansprüche

1. *In vitro* Durchmusterungsverfahren zur Beurteilung, Durchmusterung oder Auswahl einer Verbindung für die Behandlung oder Prävention einer entzündlichen Hautkrankheit, wobei besagtes Verfahren umfasst:
- Inkubation einer Zelle, die zur Expression von Nav1.9 in der Lage ist, mit der Verbindung,
- Bestimmung der Wirkung der Verbindung auf die Expression oder die Aktivität von Nav1.9, und
- Auswahl der Verbindung, wenn diese in der Lage ist, die Expression oder die Aktivität von Nav1.9 zu hemmen.

2. Verfahren gemäß Anspruch 1, wobei die entzündliche Hautkrankheit die Rosacea ist.

3. *In vitro* Verfahren zur Diagnose einer entzündlichen Hautkrankheit bei einem Patienten, der wahrscheinlich daran erkrankt ist, umfassend die Detektion und/oder die quantitative Bestimmung der Expression und/oder der Aktivität von Nav1.9 in einer biologischen Probe des Patienten.

4. Verfahren gemäß Anspruch 3, wobei die entzündliche Hautkrankheit die Rosacea ist.

5. *In vitro* Verfahren gemäß Anspruch 3 oder 4, umfassend die Schritte bestehend aus:
(a) Detektion oder quantitativer Bestimmung der Expression oder der Aktivität von Nav1.9 in einer biologischen Probe, vorzugsweise Haut, des Patienten, und
(b) Vergleich der Expression oder der Aktivität von Nav1.9, erhalten in Schritt (a), mit der Expression oder der Aktivität von Nav1.9, detektiert oder quantitativ bestimmt in einer oder mehreren biologischen Proben, vorzugsweise Haut, die von einer oder mehreren Kontrollpersonen, vorzugsweise gesunden Personen, stammen.

6. *In vitro* Verfahren zur Beurteilung der Wirksamkeit einer therapeutischen Behandlung einer entzündlichen Hautkrankheit bei einem Patienten, umfassend die Schritte bestehend aus:
(a) Detektion oder quantitativer Bestimmung der Expression oder der Aktivität von Nav1.9 in einer biologischen Probe des Patienten, vorzugsweise Haut, vor Behandlung,
(b) Detektion oder quantitativer Bestimmung der Expression oder der Aktivität von Nav1.9 in einer biologischen Probe des Patienten, vorzugsweise Haut, nach Behandlung, und
(c) Vergleich der Expression oder der Aktivität von Nav1.9 von Schritt (a) mit der in Schritt (b) bestimmten Expression oder Aktivität.

7. *In vitro* Verfahren zur Verfolgung der Progression einer entzündlichen Hautkrankheit bei einem Patienten, umfassend die Schritte bestehend aus:
(a) Detektion oder quantitativer Bestimmung der Expression oder der Aktivität von Nav1.9 in einer ersten biologischen Probe des Patienten, vorzugsweise Haut, zu einem Zeitpunkt t1,
(b) Detektion oder quantitativer Bestimmung der Expression oder der Aktivität von Nav1.9 in einer zweiten biologischen Probe des Patienten, vorzugsweise Haut, zu einem späteren Zeitpunkt t2 als Zeitpunkt t1, und
(c) Vergleich der Expression oder der Aktivität von Nav1.9 von Schritt (b) mit derjenigen von Schritt (a).

8. Verfahren gemäß einem der Ansprüche 3 bis 7, umfassend ein oder mehrere der folgenden Merkmale:
(i) die biologische Probe ist eine Dermis-Probe, eine Dermis-Epidermis-Probe oder eine Epidermis-Probe, vorzugsweise ein histologischer Schnitt, und/oder
(ii) das Verfahren umfasst die Detektion und/oder die quantitative Bestimmung der Expression von Nav1.9 in einer biologischen Probe, und/oder
(iii) die Expression von Nav1.9 wird mithilfe eines gegen Nav1.9 gerichteten Antikörpers, vorzugsweise immunhistochemisch, detektiert und/oder quantitativ bestimmt, und/oder
(iv) die entzündliche Hautkrankheit ist aus der Gruppe ausgewählt, bestehend aus Rosacea, Psorias, Pruritus, Ekzem und atopischer Dermatitis, vorzugsweise Rosacea.

9. Verfahren gemäß einem der Ansprüche 6 bis 8, wobei die entzündliche Hautkrankheit die Rosacea ist.

## Claims

1. An *in vitro* screening method for evaluating, screening or selecting a compound intended for the treatment or the prevention of an inflammatory skin disease, said method comprising:
- incubating a cell capable of expressing Nav 1.9 with the compound,
- determining the effect of the compound on the expression or the activity of Nav 1.9, and
- selecting the compound if it is capable of inhibiting the expression or the activity of Nav 1.9.

2. The method according to claim 1, wherein the inflammatory skin disease is a rosacea.

3. An *in vitro* method for diagnosing an inflammatory skin disease in a patient which may be affected therewith, comprising detecting and/or quantifying the expression and/or of the activity of Nav 1.9 in a biological sample from the patient.

4. The method according to claim 3, wherein the inflammatory skin disease is a rosacea.

5. The *in vitro* method according to claim 3 or 4 comprising the steps consisting of :
a) detecting or quantifying the expression or the activity of Nav 1.9 in a biological sample, preferably a skin sample, from the patient, and
b) comparing the expression or the activity of Nav 1.9 obtained in step (a) with the expression or the activity of Nav 1.9 detected or quantified in one or several biological samples, preferably skin samples, from one or several control subjects, preferably healthy subjects.

6. An *in vitro* method for evaluating the effectiveness of a therapeutic treatment of an inflammatory skin disease in a patient comprising the steps consisting of:
a) detecting or quantifying the expression or the activity of Nav 1.9 in a biological sample of the patient, preferably a skin sample, before treatment,
b) detecting or quantifying the expression or the activity of Nav 1.9 in a biological sample from the patient, preferably a skin sample, after treatment, and
c) comparing the expression or the activity of Nav 1.9 of step (a) with that determined at step (b).

7. An *in vitro* method for tracking the progression of an inflammatory skin disease in a patient comprising the steps consisting of:
a) detecting or quantifying the expression or the activity of Nav 1.9 in a first biological sample from the patient, preferably a skin sample, at a time t1,
b) detecting or quantifying the expression or the activity of Nav 1.9 in a second biological sample from the patient, preferably a skin sample, at a time t2 after time t1, and
c) comparing the expression or the activity of Nav 1.9 of step (b) with that of step (a).

8. The method according to any one of claims 3 to 7 comprising one or several of the following features:
(i) the biological sample is a dermis sample, a dermis-epidermis sample or an epidermis sample, preferably, a histological section, and/or
(ii) the method comprises the detection or quantification of the expression of Nav 1.9 in the biological sample, and/or
(iii) the expression of Nav 1.9 is detected and/or quantified by means of an antibody directed against Nav 1.9, preferably by immunohistochemistry, and/or
(iv) wherein the inflammatory skin disease is selected from the group consisting of rosacea, psoriasis, pruritus, contact eczema and atopic dermatitis, preferably rosacea.

9. The method according to any one of claims 6 to 8, wherein the inflammatory skin disease is rosacea.
